# EUROPEAN PATENT APPLICATION

(11) **EP 1 584 300 A2**
(43) Date of publication of application: **12.10.2005**
(21) Application number: 05251881.8
(22) Date of filing: 24.03.2005
(51) Int. Cl.: A61B 19/00

(54) **Manipulator apparatus**

(30) Priority: 30.03.2004 JP 2004101207
(71) Applicant: KABUSHIKI KAISHA TOSHIBA, Tokyo 105-8001 (JP)
(72) Inventor: Jinno, Makoto IP Division Toshiba Corporation, Minato-ku, Tokyo 105-8001 (JP); Sunaoshi, Takamitsu IP Div. Toshiba Corporation, Minato-ku, Tokyo 105-8001 (JP); Ido, Kenji IP Division Toshiba Corporation, Minato-ku, Tokyo 105-8001 (JP); Tomioka, Kei IP Division Toshiba Corporation, Minato-ku, Tokyo 105-8001 (JP)
(74) Representative: Granleese, Rhian Jane

(57) **Abstract**

A manipulator apparatus (200) comprises a surgical treatment section (213) to be inserted into a subject body, a supporting section (210) which turnably supports the surgical treatment section (213) around a first axis and a second axis, an arm section (231) having the supporting section (210) connected to a distal end side thereof, a manipulating section (220) provided at a proximal end side of the arm section (231), a manipulating lever (222) provided at the manipulating section (220), the manipulating lever (222) being turnably supported around a third axis (P) provided at the proximal end side of the arm section (231), and a manipulating device (223) provided at the manipulating lever (222), the manipulating device (223) causing the surgical treatment section (213) to turn around the first axis and the second axis.

## Description

The present invention relates to a manipulator apparatus such as a manipulator for assisting surgical operation or a manipulator for repairing a narrow part of an energy device, and more particularly, to a manipulator apparatus capable of minimizing an affect of interference caused by mutual manipulations of positioning a surgical treatment section and determining a posture; facilitating a fine manipulation and a high precision manipulation; and ensuring high operability, reliability, and safety.

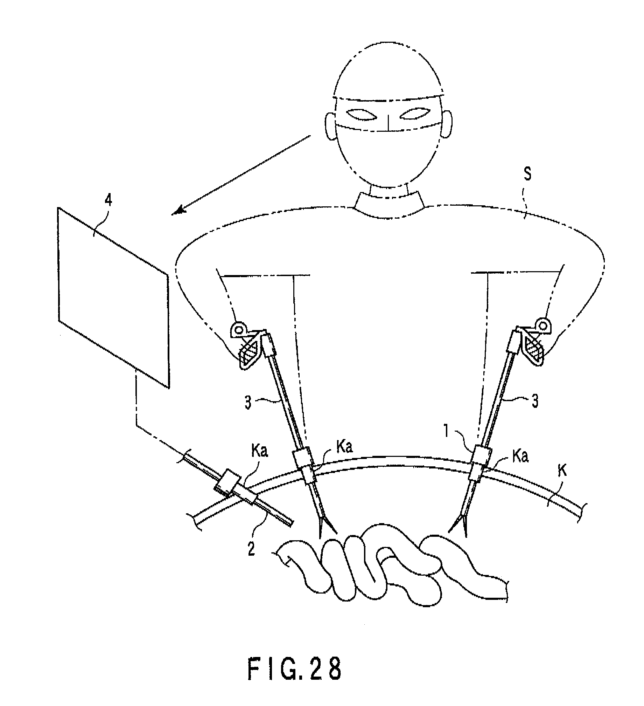

In a surgical operation under endoscopy of an abdominal cavity such as cholecystectomy, as shown in FIG. 28, some small holes "Ka" are punctured at the abdominal part of patient "K", a trocar 1 is mounted on the holes, and an endoscope 2, a forceps 3 and the like are inserted in the holes through the trocar 1. Then, an operator (in general, a surgeon) S undergoes a surgical operation while watching a video of the endoscope 2 on a monitor 4. Since such a surgical operation does not require laporotomy, a burden on a patient is reduced, and the number of days required for postoperative recovery or leaving hospital is remarkably reduced. Therefore, expansion of an applicable field is expected.

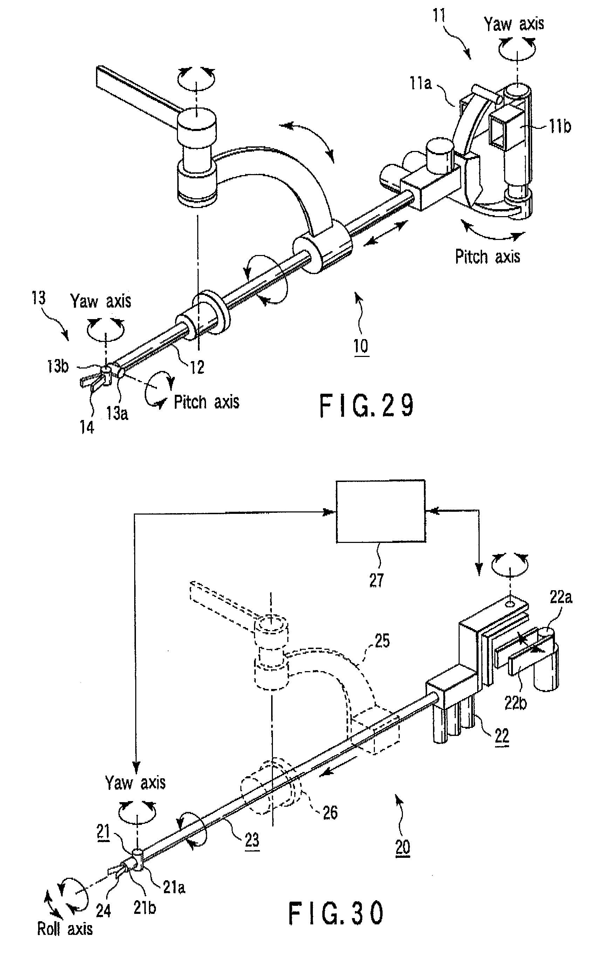

A medical manipulator (robot forceps) 10 having a robotic technology applied to a conventional forceps is known (for example, Jpn. Pat. Appln. KOKAI Publication No. 2000-350735). FIG. 29 is a perspective view showing an example of such a medical manipulator. The medical manipulator 10 comprises: an manipulation instructing section 11 having a posture manipulating section 11a and a surgical treatment manipulating section 11b; a link section 12 connected the manipulation instructing section 11 at one end thereof; a supporting section 13 connected to the other end of the link section 12, the work section having joints 13a and 13b for supporting a surgical treatment device 14 so that its posture can be changed with two degrees of freedom 2 or more; and a control section (not shown) for transmitting a manipulating instruction from the posture manipulating section 11a to the joints 13a and 13b to change the posture of the surgical treatment device 14, transmitting a manipulating instruction from the posture manipulating section 11a to the joints 13a and 13b to change the posture of the surgical treatment device 14, and transmitting a manipulating instruction from the surgical treatment manipulating section 11b to operate the surgical treatment device 14.

In addition, a medical manipulator having allocation with two degrees of freedom suitable to a suture or ligation work (for example, Jpn. Pat. Appln. KOKAI Publication No. 2002-102248). FIG. 30 is a perspective view showing an example of such a medical manipulator. A medical manipulator 20 comprises a supporting section 21; a manipulation instructing section 22; and a link section 23 connected to the supporting section 21 and the manipulation instructing section 22 at both ends thereof, wherein a surgical treatment section (gripper) 24 for treating a surgical operation part is further provided at the supporting section 21.

The supporting section 21 has: a yaw axis joint 21a and a roll axis joint 21b serving as support sections for supporting the surgical treatment section 24 so that its posture can be changed with two degrees of freedom. The manipulation instructing section 22 has: a posture manipulating section 22a for manipulating a posture with two degrees of freedom; and a surgical treatment manipulating section 22b for making an opening or closing manipulation of the surgical treatment section 24. In FIG. 30, reference numeral 25 designates a suspension device; 26 designates a trocar; and 27 designates a control circuit.

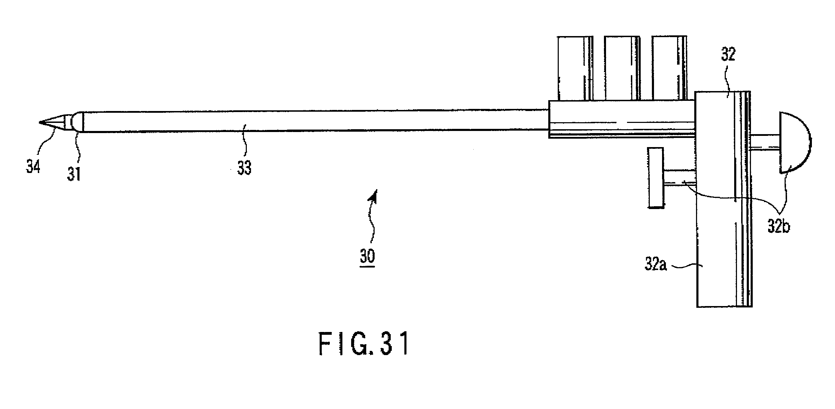

Further, a medical manipulator for independently making a positioning manipulation and a posture determining manipulation is known (for example, Jpn. Pat. Appln. KOKAI Publication No. 2001-276091). FIG. 31 is a side view showing an example of such a medical manipulator. A medical manipulator 30 comprises: a supporting section 31; a manipulating section 32; and a link section 33 connected to the work section 31 and the manipulating section 32 at both ends thereof, wherein a surgical treatment section (gripper) 34 for treating a surgical operation part is further provided at the supporting section 31. The manipulating section 32 comprises a first manipulating section 32a for positioning and a second manipulating section 32b for determining the posture of the surgical treatment section 34. The positioning manipulation and the posture determining manipulation are carried out independently. Thus, an effect of interference caused by these mutual manipulations is minimized; a fine manipulation is facilitated; and reliability and safety can be improved.

In comparison with a remote controlled master slave manipulator, the medical manipulators 10 to 30 shown in FIGS. 29 to 31 enable both of a simpler, more reliable, wider, and quicker manipulation which is achieved by linking and integrating a manipulating section (master) and a forceps distal end part hand (slave) with each other; and a fine work or a manipulation in a difficult angle which is an advantage of the manipulator. This manipulator has a joint such as bending and rotation at a distal end thereof, so that the posture of the hand can be freely moved and a suture work or a ligation work in a variety of directions which has been difficult in a conventional forceps can be facilitated. In addition, this manipulator can be used together with a conventional surgical operation device such as use of a robot forceps for the right hand and use of the conventional forceps for the left hand. Further, the system is easy and compact, thus making it possible to advantageously introduce the equipment at a low cost.

A manipulator having a similar configuration is also suitable to a work in place where it is difficult for a worker to do a work on the spot, i.e., a repair work or the like of a narrow part such as an energy device. Of course, the dimensions (length, thickness, size and the like) of the manipulator are designed according to the scope of work or a work area. Therefore, this manipulator is not always limited to a medical application.

The above-described manipulator such as the medical manipulator has suffered from the following problem. That is, the manipulator for assisting a surgical operation or the manipulator for repairing a narrow part such as an energy device requires: minimization of an effect of interference caused by mutual manipulations of positioning and posture determination when the position and posture of the surgical treatment section are induced; and easy fine manipulation and high precision manipulation. That is, high operability, reliability, and safety are required. In order to meet these requirements, it is important how to configure a manipulating section which is a direct interface. In particular, in the above-described medical manipulators 10 to 30, there is a restriction that a master and a slave are integrated with each other, and thus, the shape, dimensions, allocation and the like of the manipulating section have a great effect on high precision manipulation and operability.

The manipulating section having a configuration shown in FIGS. 29 and 30 detects the posture of the surgical treatment manipulating section of the manipulating section as a joint angle (such as a yaw axis and a pitch axis or a yaw axis and a roll axis), by means of an angle detecting sensor such as a potentiometer, and defines the detected angle as a value to be instructed to a joint angle of the supporting section. This method is similar to a master and a slave caused by a general master slave manipulator. In this case, the posture of the surgical treatment manipulating section is identical to the posture of the surgical treatment section of the supporting section, and manipulation can be intuitively carried out. Thus, the manipulating section is employed for a general master slave manipulator.

However, in the case of considering application to a surgical operation under endoscopy of an abdominal cavity, the position of a trocar cannot be changed at any time during a surgical operation. Thus, in the case where an attempt is made to induce the supporting section to a desired position, the manipulating section must often be moved in a wide range. Therefore, an operator (surgeon) must make manipulation of the right and/or left medical manipulators by widely extending one's left and right arms, In this case, in order to induce the posture of the surgical treatment manipulating section to a predetermined posture, an excessive burden is applied to the posture of the operator's wrists. In particular, in the case where the operator makes manipulation while widely extending one's left and right arms, the posture of doing a work with the distal end of the surgical treatment section being inwardly oriented (in the center direction of the left and right) applies an excessive burden on the posture of the operator's wrists. Accordingly, only a surgical operation in a narrow region in which a burden is not applied to the operator's wrists so much has been carried out. In addition, in the medical manipulators 10 to 30 or the like, if a manipulation is carried out in unnatural posture of the operator's wrists, it becomes difficult to minimize an affect of interference caused by mutual manipulations of positioning and posture determination, and it becomes impossible to carry out a fine manipulation and a high precision manipulation.

In the case of the medical manipulator 20, during suturing, a bending needle can be induced in an ideal orbit by manipulating only a roll axis. However, in a state in which an excessive burden is applied to the posture of the operator's wrists, even if an attempt is made to induce the bending needle in an ideal orbit by manipulating only the roll axis, another axis such as a yaw axis or a common roll axis moves, thus making it very difficult to induce the bending needle in an ideal orbit.

It is an object of the present invention to provide a manipulator apparatus capable of minimizing an affect of interference caused by mutual manipulations of positioning a surgical treatment section and determining a posture; facilitating a fine manipulation or a high precision manipulation; and ensuring high operability, reliability, and safety.

According to an aspect of the present invention, there is provided a manipulator apparatus comprising: a surgical treatment section to be inserted into a subject; a supporting section which supports the surgical treatment section turnably around a first axis and a second axis; an arm section having the supporting section connected to a distant end side thereof; and a manipulating section provided at the proximal end side of the arm section, wherein the manipulating section comprises: a manipulating section supported turnably around a third axis provided at the proximal end side of the arm section; and a manipulating device provided at the manipulating lever, the manipulating device causing the surgical treatment section to turn around the first axis and the second axis.

According to the present invention, an effect of interference caused by mutual manipulations of positioning and posture determination of the surgical treatment section is minimized; a burden on an operator is minimized; a fine manipulation and a high precision manipulation are easy; and a manipulation with high operability, reliability, and safety can be carried out.

This summary of the invention does not necessarily describe all necessary features so that the invention may also be a sub-combination of these described features.

The invention can be more fully understood from the following detailed description when taken in conjunction with the accompanying drawings, in which:
FIG. 1 is a perspective view showing a medical manipulator system according to a first embodiment of the present invention;
FIG. 2 is a side view showing a medical manipulator incorporated in the medical manipulator system;
FIG. 3 is a perspective view showing a supporting section incorporated in the medical manipulator;
FIG. 4 is an illustrative view illustrating a manipulating section incorporated in the medical manipulator;
FIG. 5 is an exploded perspective view showing essential portions of the manipulating section;
FIG. 6 is a block diagram depicting a configuration of a control device incorporated in the medical manipulator;
FIG. 7 is a perspective view showing a modified example of a manipulating lever incorporated in the manipulating section;
FIG. 8 is a perspective view showing a modified example of the manipulating lever incorporated in the manipulating section;
FIG. 9 is a perspective view showing a modified example of the manipulating lever incorporated in the manipulating section;
FIG. 10 is a perspective view showing a modified example of the manipulating lever incorporated in the manipulating section;
FIG. 11 is a sectional view showing essential portions of the manipulating lever;
FIG. 12 is a perspective vie showing a modified example of the supporting section incorporated in the medical manipulator;
FIG. 13 is a perspective view showing a modified example of the medical manipulator;
FIG. 14 is an illustrative view illustrating a modified example of the manipulating section incorporated in the medical manipulator;
FIG. 15 is an illustrative view illustrating a modified example of the manipulating section incorporated in the medical manipulator;
FIG. 16 is an illustrative view illustrating a modified example of the manipulating section incorporated in the medical manipulator;
FIG. 17 is an illustrative view illustrating a manipulation quantity comparative computation of the manipulating section incorporated in the medical manipulator;
FIG. 18 is an illustrative view illustrating a manipulation quantity comparative computation of the manipulating section incorporated in the medical manipulator;
FIG. 19 is an illustrative view illustrating a modified example of the manipulating section incorporated in the medical manipulator;
FIG. 20 is a side view showing a modified example of the manipulating section
FIG. 21 is a side view showing a modified example of the manipulating section
FIG. 22 is a side view showing essential portions of a medical manipulator according to a second embodiment of the present invention;
FIG. 23 is a perspective view showing a supporting section of the medical manipulator;
FIG. 24 is a side view of essential portions showing a modified example of the medical manipulator;
FIG. 25 is a side view showing essential portions of a medical manipulator according to a third embodiment of the present invention;
FIG. 26 is a side view showing essential portions of a medical manipulator according to a fourth embodiment of the present invention;
FIG. 27 is a perspective view sowing an endoscope suspension device used together with a medical manipulator according to a fifth embodiment of the present invention;
FIG. 28 is an illustrative view showing a conventional medical manipulator;
FIG. 29 is a perspective view showing the conventional medical manipulator;
FIG. 30 is a perspective view showing the conventional medical manipulator; and
FIG. 31 is a perspective view showing the conventional medical manipulator.

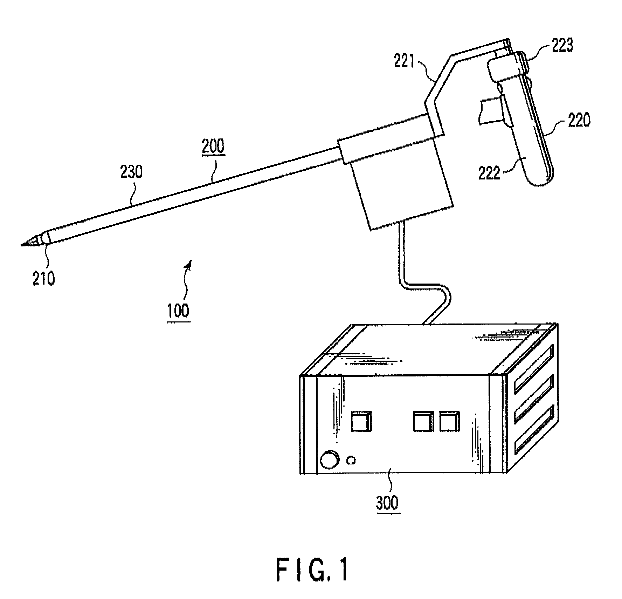

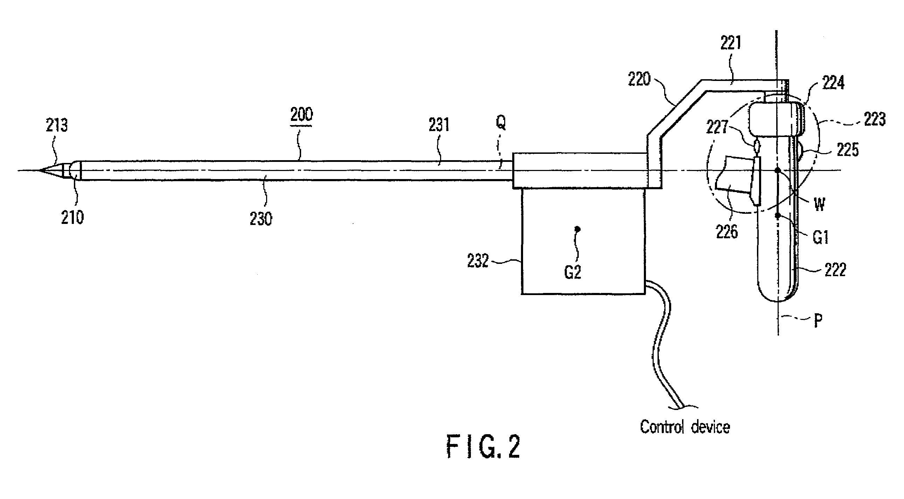

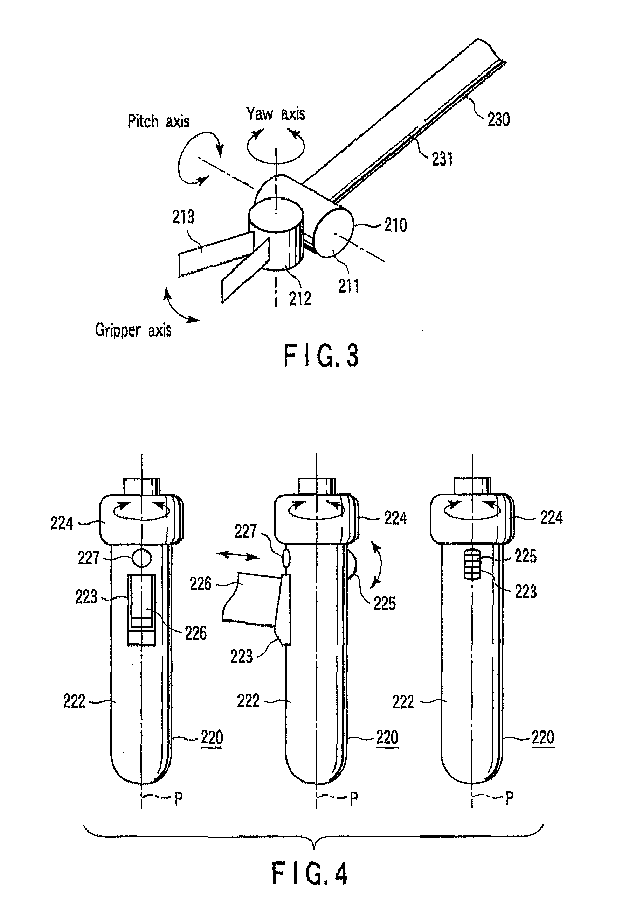

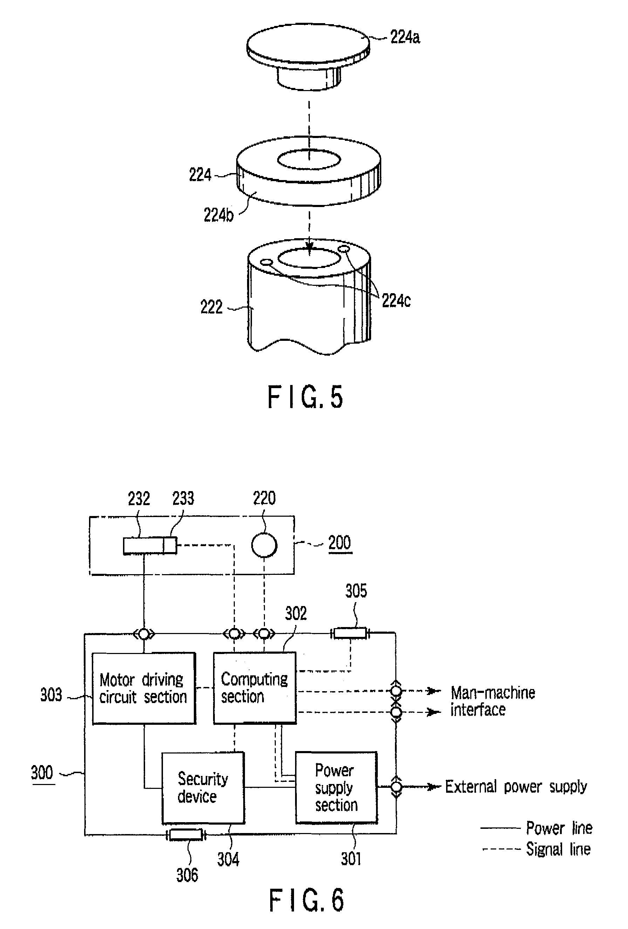

FIG. 1 is a perspective view showing a medical manipulator system 100 according to a first embodiment of the present invention; FIG. 2 is a side view showing a medical manipulator 200 incorporated in the medical manipulator system 100; FIG. 3 is a perspective view showing a supporting section 210 incorporated in the medical manipulator 200; FIG. 4 is an illustrative view illustrating a manipulating section 220 incorporated in the medical manipulator 200; FIG. 5 is an exploded perspective view showing essential portions of the manipulating section 220; and FIG. 6 is a block diagram depicting a configuration of a control device 300 incorporated in the medical manipulator 100.

As shown in FIG. 1, the medical manipulator system 100 comprises: the medical manipulator 200 which operates in a master slave system; and the control device 300 which controls and drives the medical manipulator 200.

As shown in FIG. 2, the medical manipulator 200 comprises: the supporting section 210 inserted into a cavity of the patent's body; the manipulating section 220 to be manipulated by an operator; and a link section 230 for integrally linking these elements.

The supporting section 21.0, as shown in FIG. 3, comprises: a pitch axis joint 211 mounted at the distal end side of an arm member 231 described later; a yaw axis joint 212 turnably jointed on the pitch axis joint 211 in a pitch axis (first axis) direction; and a surgical treatment device 213 turnably jointed on the yaw axis joint 212 in a yaw axis (second axis) direction. The surgical treatment device 213 is configured so as to be freely opened and closed.

The manipulating section 220 comprises: a bracket 221 connected to the proximal end side of the link section 230; a columnar manipulating lever 222 turnably mounted around a manipulating lever axis P (third axis) with respect to the bracket 221; and a manipulating device 223 mounted on the manipulating lever 222. The manipulating lever axis p which is a center axis of the manipulating lever 222 is allocated at a position crossing a center axis Q (straight line connecting a distal end and a proximal end of the arm section 231) of the arm section 231 of the link section 230. This crossing position is defined as W. The manipulating device 223 of the manipulating lever 222 has a function for determining the posture of the surgical treatment device 213.

The manipulating device 223, as shown in FIG. 4, comprises a horizontal direction dial (yaw axis manipulation: first dial) 224; a vertical direction dial (pitch axis manipulation: second dial) 225; a trigger 226 for opening and closing the surgical treatment device 213; and a manipulating mode changeover switch 227. The horizontal direction dial 224 and the vertical direction dial 225 are allocated so that manipulation can be made by a thumb. The trigger 226 and the manipulating mode changeover switch 227 are allocated so that manipulation can be made by an index finger. Of course, the horizontal direction dial 224 and the manipulating mode changeover switch 227 may be manipulated by an easily operable finger such as an index finger; the vertical direction dial 225 may be manipulated by an easily operable finger such as a thumb; and the trigger 226 may be manipulated by an easily operable finger such as a middle finger.

In such a case of a radio knife whose surgical treatment section does not require an opening or closing operation, there is no need for a degree of freedom for opening or closing and a driving system. Thus, there is no need for providing the trigger 226, for example.

The horizontal direction dial 224, the vertical direction dial 225, and the trigger 226 have sensors for detecting their turning quantities incorporated therein, and a sensor signal is processed by the control device 300.

In the horizontal direction dial 224 supported turnably around an axis on the manipulating lever axis P (first track axis) and the vertical direction dial 225 supported turnably around an axis orthogonal to the manipulating lever axis P (second track axis), an incremental encoder for detecting a turning angle is incorporated as a sensor for detecting a relative turning quantity with respect to the manipulating lever 222. The incremental encoder detects a turning quantity of a dial in a control periodic interval, and adds an angle corresponding to its detected quantity to a current target value, thereby computing a relative turning quantity with respect to the posture of the manipulating section during system startup or when a master slave manipulation starts.

The horizontal direction dial 224 and the vertical direction dial 225 are formed in a cylindrical shape in which their reference positions are not clear. Thus, a relative turning quantity of a dial from a state preceding the control periodic interval is computed after converted into a target value of the supporting section 210 by the control device 300. This eliminates inconvenience of setting the dial to the reference posture every time the manipulator is used, and improves operability.

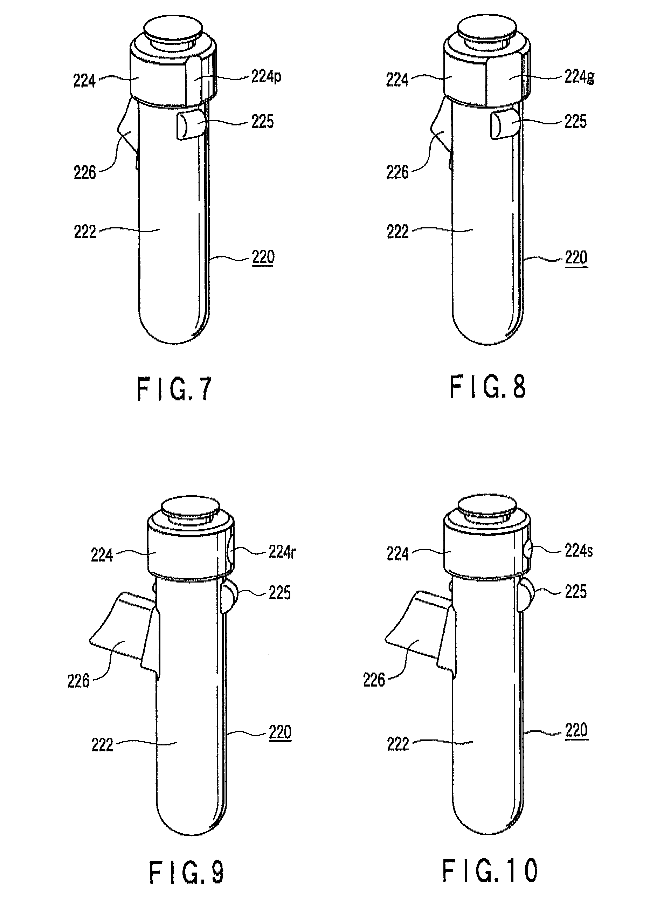

If an absolute value output sensor (such as an absolute encoder or a potentiometer) is applied for the horizontal direction dial 224 and the vertical direction dial 225, a dial section may be equipped with a reference position indicating section (reference mark or reference shape) for clarifying a reference posture. For example, as shown in FIGS. 7 to 10, the horizontal direction dial 224 is formed in a shape such that its reference position can be identified with touch. In FIGS. 7 to 10, like functional elements in FIG. 4 are designated by like reference numerals.

FIG. 7 shows a reference portion 224p formed in a linear recessed shape which is parallel to a rotation axis of the horizontal direction dial 224; FIG. 8 shows a reference portion 224q formed on a flat face; FIG. 9 shows a reference portion 224r formed in a spherical recessed shape; and FIG. 10 shows a reference portion 224s formed in a spherical recessed shape. In addition, a reference line is represented on the dial 224, whereby the mark or scale corresponding to the manipulating lever 222 may be engraved. The reference position indicating section may be allocated at a portion which can be easily visually recognized or may be allocated at a portion at which a finger for manipulating the dial is placed in natural posture (natural and neutral posture without any movement). Further, this indicating section may be allocated at a portion which is close to another dial so as to reduce a movement quantity of a manipulating finger.

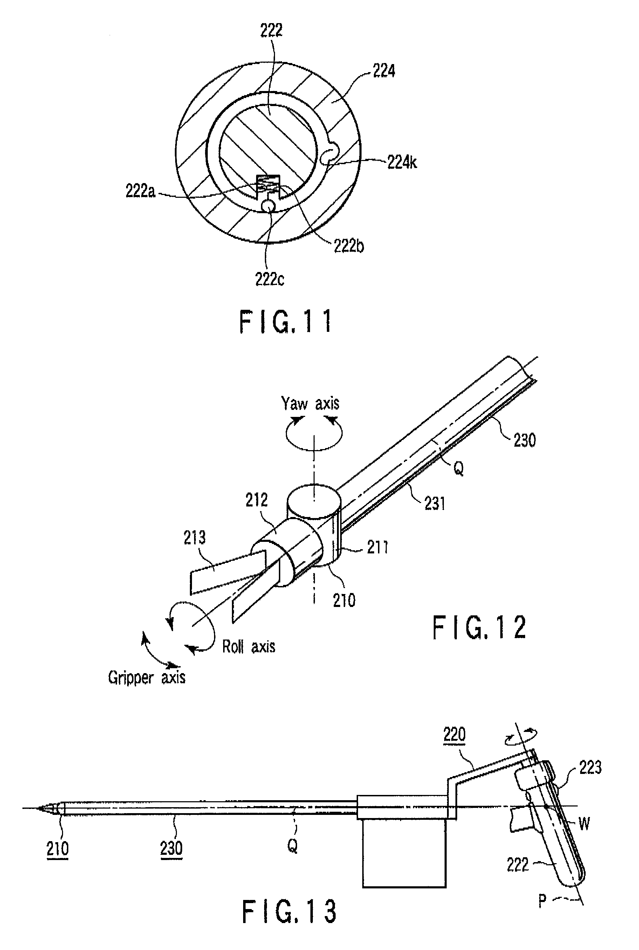

Further, in the case where the mark or reference shape coincides with a reference position, the operator may be recognized by feeling a click. In order to cause the operator to feel a click, for example, the indicating section is configured as shown in FIG. 11. That is, a recessed portion 222a is provided at the manipulating lever 222, and a proximal end portion of a spring 222b is fixed to the recessed portion 222a. Then, a sphere 222c is mounted at a distal end of the spring 222b. Also, a recessed portion 224k is provided at the horizontal direction dial 224. In this manner, when the horizontal direction dial 224 reaches a reference position, the sphere 222c is gently engaged with the recessed portion 224k, so that the operator feels a click.

Moreover, instead of ensuring that the operator feels a click, information read by a sensor can be indicated by a monitor display or by sound. In the vicinity of a turning position which generates the feeling of a click, the sphere 222c is prone to be engaged with the recess section 224k. Thus, although skillfulness is required to finely turn the dial freely, the reference position can be indicated by a monitor display or by a sound instead of a mechanism for imparting the feeling of a click. In this manner, fine adjustment of turning can be easily made by an unskilled operator.

Of course, the indication by the feeling of a click, a monitor display, and a sound can be used altogether. According to such a configuration, there is provided advantageous effect that a recognition rate of a reference posture increases, and safety is improved, for example, when a device passes through a trocar.

On the other hand, with respect to a device whose manipulation range is limited such as the trigger 226, there is provided a slide potentiometer or the like for processing a quantity detected by a sensor as an absolute value and computing a posture target value. A lock mechanism to be fixed at a bottom dead center may be provided as the trigger 226.

With respect to the above-described manipulating section 220, its structure will be described in more detail. As is evident from FIG. 4, the manipulating lever 222 is formed in a simple cylindrical external shape. Thus, this manipulating lever has a structure which can be comparatively washed. FIG. 5 is an exploded perspective view showing a sensor section of the horizontal direction dial 224. A non-contact type, reflection type optical encoder is used as the sensor section, and the above configuration is provided, whereby a system having high safety and reliability can be constructed. In FIG. 5, reference numeral 224a designates a capping section; 224b designates a wheel; and 224a designates an irradiation light receiving section. The irradiation light receiving section 224c is provided at the side of the manipulating lever 222, and a reflection face is allocated at the side of the wheel 224b, wherein a portion requiring wiring and a portion unsuitable to water wash can be allocated inside of the manipulating lever 222, and wash property can be obtained. On the other hand, the wheel 224b has a removable structure such that the dirt entering a gap between the manipulating lever 222 and the wheel 224b can be easily cleaned.

Blood or the like adheres to the operator's hands due to a surgical operation, and also, blood adheres to the medical manipulator 220 manipulated by the operator's hand. If the dial is rotationally operated in a state in which blood adheres to a manipulating dial surface, a portion at which blood adheres may spread the irradiation light receiving section 224c. Thus, two irradiation light receiving sections 224c are provided to one wheel 224b so that the two irradiation light receiving sections 224a are configured to output equal change quantities. The two irradiation light receiving sections 224c are mutually identical outputs in general. Thus, even if a change of one of these outputs stops after the scales of the output values of these two sections have been compared with each other by means of the control device 300, it is determined that manipulation is made if one output change is verified, and computation is carried out. If blood enters a gap, and adheres to the irradiation light receiving section 224c, the blood can be recognized by the other irradiation light receiving section 224c, thus making it possible to continuously use a sensor function, In addition, even if blood adheres to a part of the wheel 224b, an operation of that part is not disabled. Even if an output is provided onto one irradiation light receiving section 224c after the blood adhered part of the wheel 224b has passed, no blood adheres to the wheel 224b passing through an upper part of the other irradiation light receiving section 224c positioned distant from the irradiation light receiving section 224c, thus making it possible to normally read data. In this manner, the reliability of the manipulating section 220 can be improved.

In the case of using an absolute value output sensor such as a potentiometer whose rotation angle detecting range is limited to about 0 to 300 degrees in general, a plurality of potentiometers are allocated to be shifted each other in phase. These outputs are configured in combination so as to determine an angle, thus making it possible to eliminate limitation to a turning angle as in an incremental encoder.

The manipulating mode changeover switch 227 is provided as a switch for changing a manipulating mode such as start and end of master slave movement or initial posture restoring movement.

The link section 230 comprises: a circular tube shaped arm section 231; and a driving section 232 provided at the proximal end side of the arm section 231. The driving force generated at the driving section 232 is transmitted to the supporting section 210 via a power transmitting section (not shown) composed of a wire, a rod, a gear or the like allocated in the arm section 231.

The control device 300, as shown in FIG. 6, comprises a power supply section 301, a computing section 302, a motor drive circuit section 303, a security device 304, a variety of switches 305 and 306, and the like.

The computing section 302 has a storage device, a logic circuit, an interface, and the like. This computing section has: a function for reading a manipulation angle of the manipulating section 220 and generating a control target value of the driving section 232 for driving the supporting section 210; and a function for reading information of an angel sensor 233 for measuring a manipulation angle of the driving section 232 and comparing the control target value of the driving section 232 and computing a motor instruction input so as to eliminate its deviation. The computing section also monitors a signal input from the variety of switches 305 and 306 or the like, and carries out control or computation in accordance with a predetermined program. The computing section 302 repeats these processing operations in accordance with a predetermined control cycle.

The motor drive circuit 303 is provided as a circuit for outputting power to the driving section 232 in accordance with an instructive input from the computing section 302. The security device 304 is provided as a device for shutting out power to the medical manipulator 200 in case of an emergency such as a computing cycle failure of the computing section 302, a motor drive circuit failure, or an emergency stop instruction, and immediately stopping the operation. The variety of switches 305 and 306 are provided in a cabinet of the control device 300, and is used for, for example, a switch instruction of an operating state of the medical manipulator 200 and power supply switching.

A man-machine interface is a device provided in the cabinet of the control device 300 or connected thereto. Thus interface is used for a switching instruction of an operating state of the medical manipulator 200, power supply switching, display of a state of the operating mode, and the like.

The thus configured medical manipulator system 100 is handled as follows. The operator (surgeon) inserts the supporting section 210 into an abdominal cavity through a trocar of the patient's abdominal wall, and manipulates the manipulating section 220, thereby inducing the surgical treatment device 213 of the supporting section 210 at a predetermined position or posture and carrying out a surgical operation under endoscopy of an abdominal cavity.

The operator grips the manipulating lever 222 and widely moves the lever, thereby making it possible to induce the supporting section 210 to an arbitrary position in the abdominal cavity with the trocar being a fulcrum. Further, the posture manipulation or gripping manipulation of the surgical treatment device 213 can be carried out by the manipulating device 223.

At this time, the manipulating lever 222 is turnably jointed around the manipulating lever axis P with respect to the bracket 221. Thus, even if the manipulating lever 222 is significantly moved, the manipulating device 223 can be maintained in orientation which is always easily operable to the operator, and an excessive load is not applied to the posture of the operator's wrist. In addition, a predetermined required angle range (for example, an angle within ±90 degrees, ±30 degrees, or ±45 degrees or a symmetrical angle, for example, may be available) is made rotatable, whereby the manipulating lever 222 can be prevented from rotating at an unnecessary angle. Further, there is attained advantageous effect of preventing cable breakage or improvement of safety during trocar insertion.

Although three posture axes are generally required for posture determination of the surgical treatment device 213, only two posture axes, a yaw axis and a pitch axis, can be manipulated by the manipulating device 223. In this case as well, the arm section 231 is turned around its center axis Q, thereby making it possible to induce the surgical treatment device 213 in an arbitrary posture. At this time, the manipulating lever axis P and the center axis Q are allocated to cross each other. Thus, even in the case where the manipulating lever 222 has been rotatably jointed, the manipulating lever 222 is rotated around the center axis Q while only the manipulating lever 222 is gripped without supporting another section, thereby the operator can lead the posture of the surgical treatment device 213 easily around the center axis Q.

Conversely, the manipulating lever axis P and the center axis Q are allocated to cross each other with respect to an external force (normal driving force, moment) subjected to the surgical treatment device 213. This makes it possible to transmit such an external force to the operator via the manipulating lever 222 and prevent the supporting section 210 from being unintentionally rotated around the center axis Q.

As has been described above, according to the medical manipulator system 100 of the present embodiment, even when the operator leads the position of the supporting section 210, the manipulating device 223 can be maintained in posture which is operable to the operator. Further, the posture induction around the center axis Q of the link section 230 of the surgical treatment device 213 can be facilitated, thereby easily eliminating an effect of interference caused by mutual manipulations of positioning and posture determination. Moreover, a fining manipulation and a high precision manipulation can be carried out, and operability, reliability, and safety can be improved. The movement of a thumb and the movement of the surgical treatment section can be made coincident with each other or easily associated with each other, thus making manipulation intuitive.

The manipulating mode changeover switch 227 is provided in the manipulating device 223, whereby all the manipulations can be carried out by a single hand without making a grip change operation or a single hand grip operation and the like. Thus, operability can be improved more remarkably and an unnecessary operation can be eliminated, thus making it possible to remarkably improve safety.

In addition, when the trigger 226 is gripped up to the bottom dead center, it is locked there, so that the surgical treatment device 213 can be fixed in a closed state. This lock is released by gripping the trigger 226 again, and the surgical treatment device 213 returns to its open state. In this manner, there is no need for the operator to apply a force to the trigger 226 in a state in which the surgical treatment device 213 grips a suture needle. This makes it possible for the operator to concentrate on a posture determining manipulation or a suture manipulation and to enable safer manipulation.

As shown in FIG. 2, the grip section center G1 of the manipulating lever 222 is allocated so as to be at the side of a gravity G2 of the medical manipulator 200 from a cross position W with respect to the center axis Q and the manipulating lever axis P. In this manner, a moment around the center axis Q of the link section 230 by a mass of the driving section 232 which is comparatively great in mass is easily supported, and operability is improved.

Further, the centers of the horizontal direction dial 224, the vertical direction dial 225, the trigger 226, and the manipulating mode changeover switch 227 are provided so as to be allocated on the same plane (that is, one plane including the manipulating lever axis P) when these centers are projected in a predetermined unidirectional manner, thereby enabling manipulation by any hand irrespective of manipulation for the left hand or right hand. A restriction on the grip change of the left and right hands of the medical manipulator 200 during a surgical operation and a work of grip change between the operator and an assistant can be eliminated. As a result, the surgical operation time is reduced, and safety is improved.

Furthermore, the horizontal direction dial 224, the vertical direction dial 225, the trigger 226, and the manipulating mode changeover switch 227 are formed in a shape symmetrical to one plane including the manipulating lever axis P, thereby enabling left hand and right hand operation smoothly without any problem.

A rate between a dial manipulation quantity and a supporting section movement quantity or a movement direction can be set in accordance with the operator's discretion by an interface provided at the control device 300. At this time, a relative quantity of the computed posture manipulation quantity may be added to a posture target value in consideration with the set rate and the code assigned by orientation. A function capable of arbitrarily setting computation processing is provided to each of the horizontal direction dial 224 and the vertical direction dial 225, thus enabling manipulation using the same manipulating section 220 even in the case where a posture axis configuration of the supporting section 210 is different from another one. For example, as shown in FIG. 12, in the case where the degrees of freedom around the yaw axis and roll axis are configured at the supporting section 220, the horizontal direction dial 224 and the vertical direction dial 225 may be assigned to the yaw axis manipulation and roll axis manipulation, respectively.

During suturing, a bending needle can be induced in an ideal orbit by manipulating only the roll axis. In the case of the manipulator according to the embodiment, however, only the vertical direction dial 225 is moved by thumb manipulation, thereby making it possible to manipulate only the roll axis. As a result, the bending needle can be induced in an ideal orbit.

Further, the horizontal direction dial 224 and the vertical direction dial 225 may be changed in size as required. That is, the movement range or movement speed around the yaw axis or roll axis at the supporting section 210 is not always identical depending an allocation of the degree of freedom of a joint, the main scope of work, or the like. For example, when a suture work is carried out, it is necessary to move a wide range of angle around the roll axis. Thus, the roll axis is assigned to the vertical direction dial and its radius is downsized, thereby making it possible for the operator to move the roll axis in a wide range of angle by slight manipulation. As a result, the efficiency of the suture work can be improved.

The medical manipulator system 100 is not limited to the above-described embodiments. For example, as shown in FIG. 13, a similar advantageous effect can be attained even if the system is allocated so that the center axis G and the manipulating lever axis P are not orthogonal to each other.

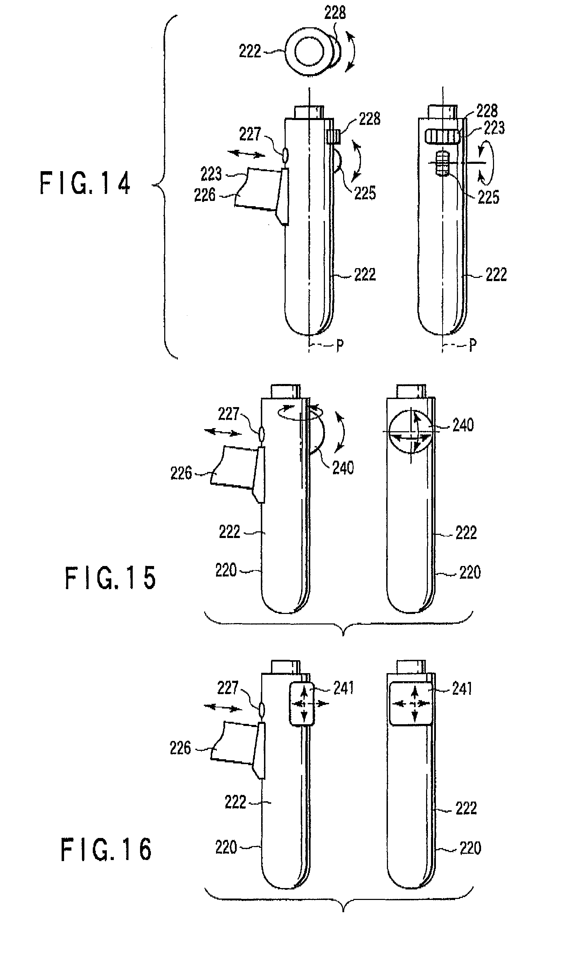

FIG. 14 is a view showing a modified example of the manipulating section 220 incorporated in the above-described medical manipulator system 100. In FIG. 14, like functional elements in FIG. 4 are designated by like reference numerals, and detailed description is omitted here.

In the manipulating section 220 shown in FIG. 14, a small sized horizontal direction dial 228 supported turnably around an axis parallel to the manipulating lever axis P (first track axis) is used instead of the horizontal direction dial 224. A similar advantageous effect can be attained in this modified example as well. In particular, as is the case where a manipulator composed of the yaw axis and the pitch axis as shown in FIG. 3 is instructed by the respective dials, the present invention is effective in the case of the system in which the manipulation quantities of these axes are equal to each other.

FIG. 15 is a view showing a modified example of the manipulating section 220 incorporated in the above-described medical manipulator system 100. In FIG. 15, like functional elements in FIG. 4 are designated by like reference numerals, and a detailed description is omitted here.

In the manipulating section 220 shown in FIG. 15, a track ball 240 capable of instructing and inputting a position at the same time in two directions is used instead of the horizontal direction dial 224 and the vertical direction dial 225. In the present modified example, there can be attained an advantageous effect similar to that in a case of using the horizontal direction dial 224 and the vertical direction dial 225. In addition, finger movement between the horizontal direction dial 224 and the vertical direction dial 225 is eliminated, thus making it possible to reduce the movement of manipulating fingers to the minimum.

In order to improve operability, a change rate of a position instructing value due to a transverse direction rotation and a longitudinal direction rotation of the track ball 240 is detected, these change rates are compared with each other, and priority is given to the position instructing value indicating a greater change rate. Accordingly, the yaw axis and the pitch axis or the yaw axis and the roll axis can be moved independently, respectively, thus making it possible to improve safety more remarkably. In the case of such a manipulating instruction for inducing a position, a movement time can be reduced, so that a method for generally moving two directions at the same time may be employed.

The operation of comparing change rates of two axis is applicable not only the case of using the track ball 240, but also the case of using the horizontal direction dial 224 and the vertical direction dial 225, and any other cases comprising rotatable device(s) which have two track axes to rotate.

In the manipulating section 220 shown in FIG, 16, a touch pad 241 capable of instructing and inputting a position at the same time in two directions is used instead of the horizontal direction dial 224 and the vertical direction dial 225. That is, finger movement in the transverse direction on the touch pad 241 is associated with the horizontal direction dial 224, and finger movement in the longitudinal direction is associated with the vertical direction dial 225, whereby there can be attained an advantageous effect similar to that in a case of using the above track ball 240.

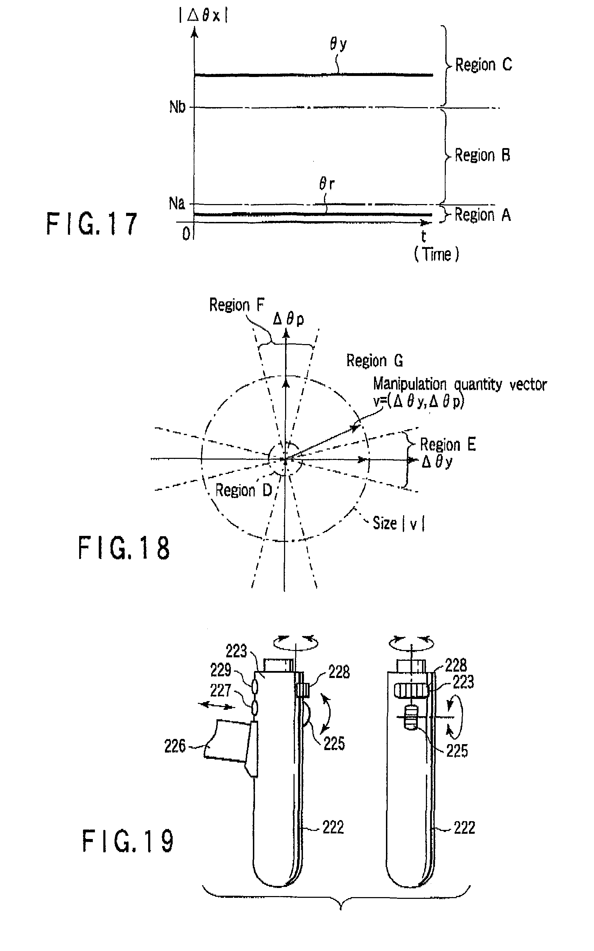

Now, with reference to FIG. 17, a description will be given with respect to a method for carrying out a transverse direction manipulation and a longitudinal direction manipulation mutually independently in order to avoid unintentional movement. For example, in a manipulation quantity θy of the yaw axis and a manipulation quantity θr of the roll axis, the target value computation is computed by comparison between the sizes of these axes and by comparison between each of the values and a reference value. Specifically, the following technique is used.

An absolute value |Δθx| (x = y, r) of a change rate of a manipulation quantity is computed every predetermined detection period of the manipulation quantity (equal to the control period, for example). When the absolute value of the change rate of the manipulation quantity is smaller than a reference value Na which is close to zero, it means that this manipulation is not intended by the operator. It is determined that a change due to a handshake Or signal noise occurs. In this case, the manipulation quantity is not added to the posture target value (region A). Next, a reference value Nb which is greater than a reference value Na is compared with an absolute value of the change rate. In the case where the absolute values of their manipulation quantities are greater than the reference value Nb, their manipulation quantities are determined to be a manipulation intended by the operator, and both of the manipulation quantities are added to the posture target value (region C). Further, the absolute values of their manipulation quantities are assumed to be greater than the reference value Na and a range smaller than Nb is defined as region B. In region B, their values are compared with each other, a greater value is added to the target value, and a smaller value is not added. In the case where the change rate in this region B is small, it is determined that such manipulation is not intended by the operator.

If one of them exists in region C and the other exist in region B, the value of region C is accepted as is, and the value of region B is not accepted. However, if one of them is the value of region B, but the other is in region A or zero, even the value of region B is accepted. This is because slow manipulation can be determined. In the case where both of the above values are in region A, these values are not accepted. In the case where an attempt is made to carry out very slow manipulation as in region A or in the case where an attempt is made to carry out a plurality of manipulations at the same time in region B, the set rate during target value conversion may be changed. Thus, in a change of a manipulation quantity, a function of switching computation of manipulation quantities according to the above-described three regions is valid. Further, a function capable of setting the size of the reference value Na or reference value Nb is provided, whereby a manipulating section according to the operator's preference can be provided, which contributes to the improvement of operability.

As has been described above, a configuration is provided so that a change rate of a position instructing value due to the transverse direction manipulation and longitudinal direction manipulation is detected; the change rates are compared with each other, and priority is given to an instructing value indicating a greater change rate according to the scale of the change rate. With this configuration, it becomes possible to move only the direction which the operator attempts to move, and a precise and sate manipulation can be achieved. Such determination may be utilized in the case of using the horizontal direction dial 224 and the vertical direction dial 225

Further, with reference to FIG. 18, a description will be given with reference to a method for computing the manipulation quantity instructing values in two directions in comparison with the target value, which is different from the method described previously. The change rates of the manipulation quantities obtained every predetermined detection cycle of manipulation quantities (equal to the control cycle, for example) can be handled as a vector. For example, the change rates in the yaw axis direction and pitch axis direction are defined as Δθy and Δθp, respectively, and the size of the obtained vector is defined as |v|. In the case the vector size is smaller than a predetermined reference value, it is determined that a change due to signal noise or hand shake occurs, and a zero vector is defined so as not to add the value to the target value (region D). In the case where the above vector size is greater than the reference value and a manipulation quantity vector falls under region E in the vicinity of the Δ0y axis, the current vector is replaced with a Δθy directional vector having a size |v|, and target value computation is carried out. Similarly, in the case where the manipulation quantity vector falls under region F in the vicinity of the Δθp axis, the current vector is replaced with a Δθp directional vector having a size |v|, and target value computation is carried out. As a result, changes of the position instructing values in two directions are compared with each other. That is, of course, the vector is computed, and a position instructing value indicating a greater change rate is transmitted with higher priority. In addition, an instructing value of a size which is proportional to a manipulation quantity can be transmitted regardless of a manipulating direction. By carrying out such computation, an unintentional two-axis manipulation is prevented, enabling an independent manipulation of only one axis. Of course, region D which is not included in the above region E and region F is set, thereby enabling an intentional two-axis simultaneous manipulation. If the boundary between region E and region F is set so that region G is eliminated, two-axis simultaneous manipulation can be completely prevented. By providing means capable of adjusting this region setting at the control device 300 or manipulating section 220, there can be provided a manipulation according to surgical operation or the configured degree of freedom of a manipulator to be used, and the operator specific habit.

FIG. 19 is a view showing a modified example of the manipulating section 220. The manipulating lever 222 incorporates a lock mechanism capable of fixing the bracket 221 at an arbitrary or predetermined angle. Lock and release can be switched by a lock switch 229 provided in the manipulating device 223. In this manner, during manipulation, the lever is rotatable; at the end of manipulation, an angle is fixed; or alternatively, when the manipulating lever 222 is induced, the lever is rotatable, and when the lever is manipulated at a substantially constant position, the lever is fixed. In this manner, the manipulating lever can be selectively rotatable and fixed according to the contents of manipulation, and the whole operability can be improved.

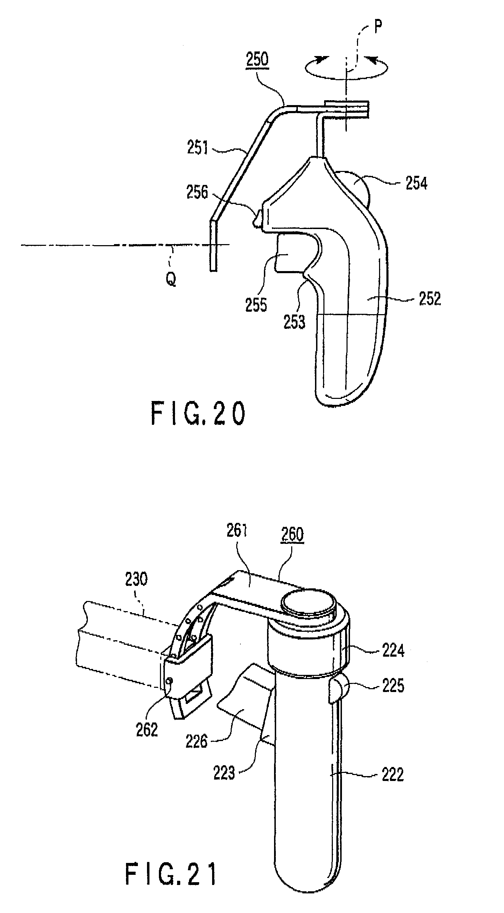

FIG. 20 is a side view showing a manipulating section 250 according to a modified example of the manipulating section 220. The manipulating section 250 comprises: a bracket 251 connected to the proximal end side of the link section 230; a manipulating lever 252 turnably mounted around the manipulating lever axis P with respect to the bracket 251; and a manipulating device 253 mounted on the manipulating lever 252. The manipulating lever axis P which is the center axis of the manipulating lever 252 is allocated at a position crossing the center axis Q of the arm section 231 of the link section 230.

The manipulating lever 252 has a function for positioning the supporting section 210, and the manipulating device 253 has a function for determining the posture of the surgical treatment device 213. The manipulating lever 252 is formed in association with the shape of a hand. In particular, a track ball 254 to be described later is allocated on an inclined face.

The manipulating device 253 comprises: the track ball 254 capable of carrying out the transverse direction manipulation (yaw axis manipulation) and the longitudinal direction manipulation (pitch axis manipulation) at the same time; a trigger 255 for opening or closing the surgical treatment device 213; and a manipulating mode changeover switch 256. The track ball 254 is allocated so as to enable manipulation by a thumb, and the trigger 255 and the manipulating mode changeover switch 256 are allocated so as to enable manipulation by an index finger.

The track ball 254 and the trigger 255 each incorporate a sensor for detecting their movement quantity, and the control device 300 processes a sensor signal.

For example, a sensor used for the track ball 254 incorporates a photo sensor which detects a relative movement quantity in two directions. Specifically, the photo sensor computes the two-axis direction component of a relative displacement of the track ball and the sensor in a control periodic interval, adds the computed component to the target value, and computes a relative quantity of the posture displacement of the manipulating section during system startup or when master slave movement starts. The track ball 254 is formed in a spherical shape in which a reference position is not clear. Thus, the posture manipulation quantity is read in a relative quantity value, and the read value is converted and computed into the target value of the supporting section 210 by the control device 300. In this manner, operability is improved by eliminating inconvenience of adjusting the track ball 254 to the reference posture every time the manipulator is used. In order to improve noise proofing property of the sensor signal, a senior output is converted into a serial signal by means of a converter circuit situated in the vicinity of the photo sensor inside of the manipulating section 250, and the converted serial signal is transmitted to the control device 300.

According to the thus configured manipulating section 250, since the position of the track ball 254 is allocated on the inclined face of the manipulating lever 252, a burden on a finger, a hand, or a wrist is reduced in a state in which the manipulating lever 252 is gripped, and the operability of the track ball 254 can be improved more remarkably. As described previously with reference to FIG. 5, of course, the movement of the track ball corresponding to one reflection plate is read by the photo sensor corresponding to a plurality of light receiving sections, and the read signals are compared with each other, thereby making it possible to improve safety and reliability. In addition, the track ball is configured to be removable from the manipulating lever 25, and the track ball contaminated during manipulation can be washed as required, and can be reused.

The other manipulating devices include a joystick, a force detecting sensor, a cross key, and a cross button. For example, with respect to the joystick, the detected angle may be replaced with a speed instruction; with respect to the force detecting sensor, the detected force may be replaced to a speed instruction parallel to the force; and with respect to the cross key or the cross button, the detected direction may be replaced with a predetermined speed instruction. In addition, the force detecting sensor has an advantage that an instruction is supplied by force detection, thus making it possible to minimize the finger movement itself. In a variety of these manipulating devices, the method described in FIG. 17 or FIG. 18 described previously can be used in processing for conversion between the manipulation quantity and the target value.

In a stick manipulation using the joystick or the force detecting sensor, the degree of freedom such as a stick twisting manipulation or a push-pull manipulation for the manipulating section is added to moving the stick longitudinally or to transversely. Thus, the setting change becomes more bending according to the manipulator configuration on the degree of freedom or the degree of freedom level. For example, in the case of joystick manipulation, the forward and backward manipulation can be assigned to the pitch axis manipulation, the left and right manipulation can be assigned to the yaw axis manipulation, and the twisting manipulation can be assigned to the roll axis manipulation.

In the stick manipulation using the force detecting sensor, the force detecting sensor is mounted at the base of the stick, and the force generated when the stick is inclined forwardly and backwardly or to the left and right is converted into a manipulation quantity. Similarly, the force detecting sensor can detect the twisting manipulation as a torque and convert the detected torque into a target value. Further, with respect to the force in the push-pull direction, a change of the force corresponding to this manipulation can be converted into a manipulation quantity of a new degree of freedom. In addition, signal processing can be carried out as a substitute of a switch signal such as mode switching. Furthermore, from a difference between the force applied by the operator during manipulation touching the stick and a detection quantity in a state in which the operator does not touch the stick, it is possible to recognize a difference between an intentional manipulation and a stick movement caused by device tilting. When it has been successfully determined that the operator does not touch the stick or that the touching force is weak, another axis force in that state and a torque input are inhibited from being converted into target values, thereby making it possible to prevent an unintentional movement.

FIG. 21 is a perspective view sowing a manipulating section 260 according to a modified example of the manipulating section 220. In FIG. 21, like functional elements in FIGS. 2 and 4 are designated by like reference numerals, and a detailed description is omitted here.

The manipulating section 260 comprises a bracket 261 connected to the proximal end side of the link section 230 at one end side thereof. The bracket 261 is secured to the link section 230 by a screw 262. By mounting and removing this screw, a fixed position of the bracket can be changed in a predetermined angle range. Of course, the fixing work may be carried out with one touch by utilizing a pin etc., or may be fixed to a continuous arbitrary position.

A columnar manipulating lever 222 turnably mounted around the manipulating lever axis P and a manipulating device 223 mounted on the manipulating lever 222 are provided at the other end of the bracket 261.

With such a configuration, the operator can grip the manipulating lever 222 at an optimal angle position, thus making it possible to improve work efficiency more remarkably.

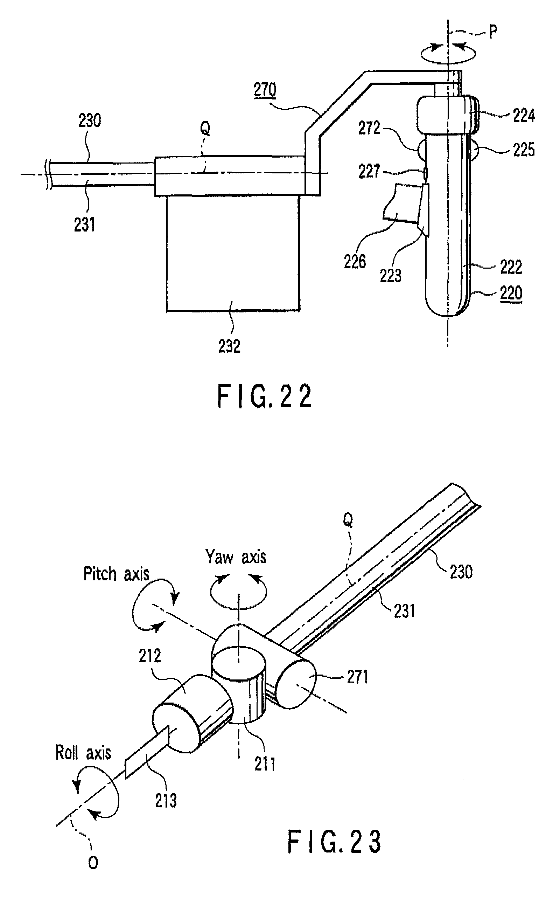

FIGS. 22 and 23 are views showing essential portions of a medical manipulator 270 according to a second embodiment of the present invention. In FIGS. 22 and 23, like functional elements in FIGS. 2, 4 and 12 are designated by like reference numerals, and a detailed description is omitted here.

The medical manipulator 270 comprises: a roll axis joint 271 mounted at the distal end side of the arm member 231; the pitch axis joint 211 turnably supported by the roll axis joint 271 in the roll axis direction; the yaw axis joint 212 turnably supported by the pitch axis joint 211 in the pitch axis (first axis) direction; and the surgical treatment device 213 turnably jointed by the yaw axis joint 212 in the yaw axis (second axis) direction. The surgical treatment device 213 is openably configured. The surgical treatment device such as a radio knife does not require any opening or closing operation.

The manipulating section 220 comprises: the bracket 221 connected to the proximal end side of the link section 230; the columnar manipulating lever 222 turnably mounted around the manipulating lever axis P with respect to the bracket 221; and the manipulating device 223 mounted on the manipulating lever 222. The manipulating lever axis P which is the center axis of the manipulating lever 222 is allocated at a position crossing the center axis Q of the arm section 231 of the link section 230. The manipulating lever 222 has a function for positioning the supporting section 210, and the manipulating device 223 has a function for determining the posture of the surgical treatment device 213.

The manipulating device 223 comprises: the horizontal direction dial (yaw axis manipulation: first dial) 224; the vertical direction dial (pitch axis manipulation: second dial) 225; a roll direction dial (roll axis manipulation: third dial) 272; the trigger 226 for opening or closing the surgical treatment device 213; and the manipulating mode changeover switch 227. The horizontal direction dial 224 and the vertical direction dial 225 are allocated to enable thumb manipulation; and the roll direction dial 272, the trigger 226, and the manipulating mode changeover switch 227 are allocated to enable index finger manipulation. Of course, the horizontal direction dial 224 and the manipulating mode changeover switch 227 may be manipulated by an index finger, the longitudinal dial 225 may be manipulating by a thumb, and the trigger 226 may be manipulating by a middle finger.

The horizontal direction dial 224, the vertical direction dial 225, the roll direction dial 272, and the trigger 226 incorporate sensors for detecting their turning quantity and movement quantity, respectively, and the control device 300 processes a sensor signal. The sensors used for the horizontal direction dial 224, the vertical direction dial 225, and the roll direction dial 272 each incorporate an incremental encoder for detecting an angle.

According to the second embodiment, the posture of the surgical treatment device 213 can be controlled in three-axis directions including the yaw axis, pitch axis, and roll axis, thus making it possible to improve operability in a finer work.

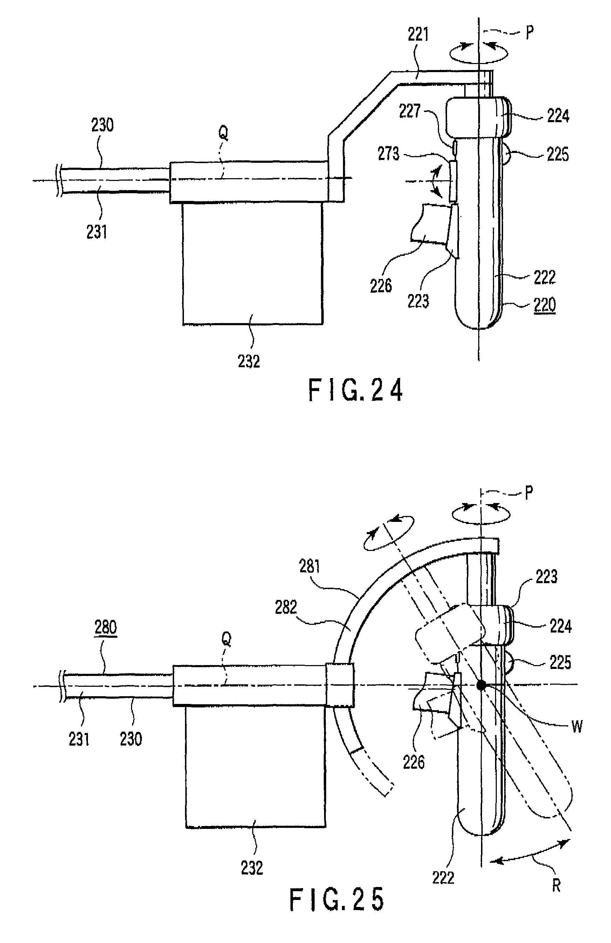

FIG. 24 is a view showing a modified example of the medical manipulator 270. In FIG. 24, like functional elements in FIG. 22 are designated by like reference numerals, and w detailed description is omitted here.

In this modified example, a seesaw switch 273 is used instead of the roll direction dial 272. In this modified example as well, there can be attained an advantageous effect similar to that in a case of using the roll direction dial 272.

In the case of a radio knife whose surgical treatment section does not require any opening or closing operation, there is no need for a degree of freedom for opening or closing and a driving system are not require, and it is not necessary to allocate the trigger 226 at the manipulating section. Alternatively, the trigger 226 is utilized as a changeover switch, whereby there may be switched between a case of assigning the horizontal direction dial 224 and the vertical direction dial 225 to the yaw axis manipulation and the pitch axis manipulation and a case of assigning the horizontal direction dial 224 and the vertical direction dial 225 to the roll axis direction manipulation. Of course, a track ball may be assigned instead of the horizontal direction dial 224 and the vertical direction dial 225. The trigger 226 is utilized as a changeover switch, thereby enabling manipulations of a plurality of supporting sections at the same manipulating section.

FIG. 25 is a side view showing essential portions of a medical manipulator 280 according to a third embodiment of the present invention. In FIG. 25, like functional elements in FIG. 2 are designated by like reference numerals. A detailed description is omitted here.

The medical manipulator 280 comprises: the supporting section 210 to be inserted into the patient's body cavity; a manipulating section 281 to be manipulated by an operator; and the link section 230 for integrally linking the sections with each other.

The manipulating section 281 comprises: an arc shaped bracket 282 slidably connected to the proximal end side of the link section 230 in its peripheral direction (in the direction indicated by the arrow R in FIG. 25); the columnar manipulating lever 222 turnably mounted around the manipulating lever axis P with respect to the bracket 282; and the manipulating device 223 mounted on the manipulating lever 222.

The manipulating lever axis P which is the center axis of the manipulating lever 222 is allocated at a position crossing the center axis Q of the arm section 231 of the link section 230. This cross position is defined as W. The arc center (fourth axis) of the bracket 282 is situated at the cross position W, and is turnable in the direction indicate by the arrow W in FIG. 25.

According to the thus configured medical manipulator 280, the manipulating lever 222 is turnable with respect to the direction indicated by the arrow R in FIG. 25 as well, and the operator can grip the manipulating lever 222 at a more optimal angle position, thus making it possible to improve work efficiency more remarkably.

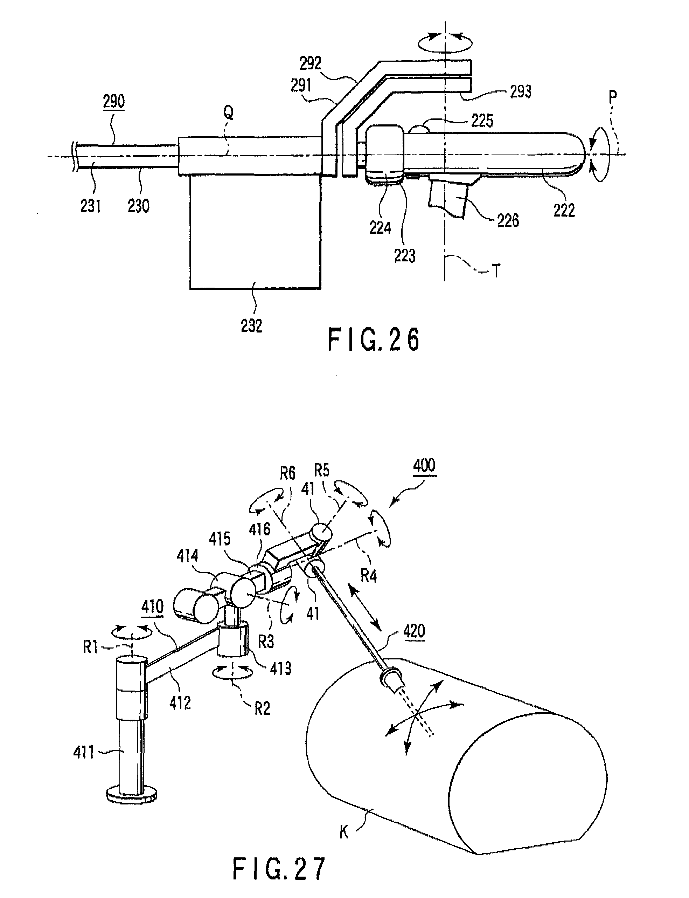

FIG. 26 is a side view showing essential portions of a medical manipulator 290 according to a fourth embodiment of the present invention. In FIG. 26, like functional elements in FIG. 25 are designated by like reference numerals. A detailed description is omitted here.

The medical manipulator 290 comprises: the supporting section 210 to be inserted into the patient's body cavity; a manipulating section 290 to be manipulated by the operator; and the link section 230 for integrally linking the sections with each other.

The manipulating section 291 comprises: a first bracket 292 connected to the proximal end side of the link section 230; a second bracket 293 turnably mounted around an axis T; the colmnnar manipulating lever 222 turnably mounted on the second bracket 293 around the manipulating lever axis P; and the manipulating device 223 mounted on the manipulating lever 222.

The manipulating lever axis P which is the center axis of the manipulating lever 222 may coincide with the center axis Q of the arm section 231 of the link section 230 by the turning angle around the axis T. However, the rotational movement of the arm section 231 around the center axis Q is limited by a self weight of a driving motor or cables and the like. Of course, the orientation of the first bracket 292 may be mounted in a direction rotated by 90 degrees with respect to the axis Q.

According to the thus configured medical manipulator 290, the operator can grip the manipulating lever 222 at a more optimal angle position, thus making it possible to improve work efficiency more remarkably.

FIG. 27 is a perspective view showing an endoscope suspension device (another device) 400 used in combination with the medical manipulator 100 according to a fifth embodiment of the present invention. Uppercase letter K in the figure designates a patient's body cavity.

The manipulating device 223 can carry out manipulation of the endoscope suspension device 400 as shown in FIG. 27 by switching a manipulating mode. In switching of the manipulating mode, a manipulating mode changeover switch 227 provided at a second manipulating section may be used or an additional footswitch or the like may be provided. In addition, a system for displaying and notifying the manipulating mode may be provided.

The endoscope suspension device 400 comprises a robot arm 410 mounted on a floor face; and an abdominal cavity endoscope 420 mounted on the robot arm 410

The robot arm 410 comprises: a first rotation axis section 411 having an axis in a vertical direction; a first arm 412 fixed to the output axis side of the first rotation axis section 411; a second rotation axis section 413 having an axis in a vertical direction; a third rotation axis section 414 mounted on the second rotation axis section and having an axis in a horizontal direction; a second arm 415 fixed at the output axis side of the third rotation axis section 414; a fourth rotation axis section 416 rotatably jointed so that the longitudinal direction of the second arm 415 becomes a rotation axis; a fifth rotation axis 417 rotatably jointed in a direction orthogonal to the fourth rotation axis section 416; and a sixth rotation axis 418 rotatably jointed in a direction orthogonal to the fifth rotation axis.

Joint driving actuators are provided at the first rotation axis section 411, the second rotation axis section 413, the third rotation axis section 414, and the sixth rotation axis 418, respectively. The sixth rotation axis section 418 may be fixed at a predetermined angle. In addition, the fourth and fifth rotation axes 416 and 417 may comprise a joint driving actuator even for a passive joint.

The thus configured endoscope suspension device 400 can be manipulated from the manipulating device 223 at the side of the medical manipulator 100, whereby the endoscope suspension device 400 can be manipulating without the help of an assistant. That is, the maintained abdominal cavity endoscope is induced to an arbitrary position. That is, an endoscope image in an arbitrary field of view can be displayed on a monitor.

In addition, the horizontal direction dial 224 can be used for movement in a transverse direction; the horizontal direction dial 225 can be used for movement in a vertical direction; and the trigger 226 can be used for movement in a longitudinal direction. An optical inducing movement (for example, zooming manipulation) as well as a mechanical inducing movement may be carried out.

According to the present embodiment, a simple system configuration including peripheral devices can be provided without requiring a new operating interface for another device such as an endoscope suspension device. Further, the operator oneself can handle the endoscope image or another device, thus, enabling one's own intentional manipulation. Moreover, there is an advantage that a surgical operation by a small number of surgeons or a surgical operation by a single surgeon (solo surgery) can be carried out, making it possible to solve a problem with an insufficient number of surgeons or the like.

In the case where a surgical treatment section is a radio knife, there is no need for providing the surgical treatment section at the manipulating lever because the radio knife is generally provided as an optional system and manipulation is often carried out by a footswitch. Of course, a surgical treatment manipulating section (for example, trigger) is provided at the manipulating lever, whereby an instruction may be sent to the radio knife system side.

Moreover, the surgical treatment section not only denotes a gripper or a radio knife, but also encompasses a CCD camera or an illumination system and the like. In addition, movable actions include a bending action or an optical action and the like. That is, the present application is easily applied to an abdominal cavity endoscope which can be bent at a distal end (flexible endoscope), making it possible to provide an abdominal cavity endoscope (flexible endoscope) with a good operability. In this case, the bending action is configured to drive a motor in the same manner as in a manipulator. Of course, it is possible to apply an optical manipulation of moving a field of view (vertical and horizontal movements) instead of the bending action.

## Claims

1. A manipulator apparatus (200) **characterized by** comprising:
a surgical treatment section (213) to be inserted into a subject body;
a supporting section (210) which turnably supports the surgical treatment section (213) around a first axis and a second axis;
a manipulating section (220) provided at a proximal end side of the arm section (231);
an arm section (231) which connects the supporting section (210) and the manipulating section (220);
a manipulating lever (222) provided at the manipulating section (220), the manipulating lever (222) being turnably supported around a third axis (P) provided at the proximal end side of the arm section (231) ; and
a manipulating device (223) provided at the manipulating lever (222), the manipulating device (223) causing the surgical treatment section (213) to turn around the first axis and the second axis.

2. A manipulator apparatus (200) according to claim 1, **characterized in that** the third axis (P) is allocated to cross a straight line connecting the distal end and the proximal end of the arm section (231) with each other.

3. A manipulator apparatus (200) according to claim 2, **characterized in that** the manipulating lever (222) is turnably supported around a fourth axis provided at the proximal end the of the arm section (231), and
the fourth axis is allocated to cross a straight line connecting the distal end and the proximal end of the arm section (231) with each other.

4. A manipulator apparatus (200) according to claim 1, **characterized in that** the manipulating device (223) is provided in plurality at the manipulating lever (222), and said plurality of manipulating devices (223) are allocated on one plane including the third axis (P).

5. A manipulator apparatus (200) according to claim 1, **characterized in that** the manipulating device (223) includes: a first dial (224) corresponding to a turning movement around the first axis of the supporting section (210) ; a second dial (225) corresponding to a turning movement around the second axis of the supporting section (210); and a trigger (226) corresponding to a surgical treatment movement of the surgical treatment section (213) .

6. A manipulator apparatus according to claim 5, **characterized in that** a reference position indicating section is formed at least one of the first dial (224), the second dial (225), and the trigger (226).

7. A manipulator apparatus (200) according to claim 5, **characterized in that** the first dial (228) and the second dial (224) are different from each other in size.

8. A manipulator apparatus (200) according to claim 4, **characterized in that** at least one of the manipulating devices (223) has a reflection plate (224b) and a plurality of irradiation light receiving sections (224c) allocated to the reflection plate (224b).

9. A manipulator apparatus (200) according to claim 1, **characterized by** comprising a fixing section (229) which fixes a turning position of the manipulating lever (222) to the third axis (P).

10. A manipulator apparatus (200) according to claim 2, **characterized in that** a center part of the manipulating lever (222) in the third axis (P) direction is allocated in proximity to a gravity of the manipulator apparatus (200) rather than a cross point between a straight line connecting the distal end and the proximal end of the arm section (231) and the third axis (P) direction.

11. A manipulator apparatus (200) according to claim 1, **characterized in that** the manipulating device (223) has a device (240, 241) which corresponds to a turning movement around the first axis and a turning movement around the second axis, of the supporting section (210).

12. A manipulator apparatus (200) according to claim 1, **characterized by** comprising:
a rotatable device (240) which rotates on a first track axis and a second track axis;
a compare section (302) which compares change rates of position indicating values of turning movements around the first track axis and the second track axis with each other, and outputs the greater change rate of position indicating values of turning movements around the first track axis and the second track axis; and
a driving section (302) which generates a driving force transmitted to the supporting section (210) according to the change rate outputted from the compare section (302).

13. A manipulator apparatus (200) according to claim 12, **characterized in that** the compare section (302) compares a predetermined reference value to the greater change rate of position indicating values of turning movements around the first track axis and the second track axis, and outputs the greater value of them when the greater value of them is greater than the predetermined reference value.

14. A manipulator apparatus (200) according to claim 10, **characterized in that** a rate of a turning movement with respect to the instruction for the turning movement around the first axis and the instruction for the turning movement around the second axis is different depending on a turning direction.

15. A manipulator apparatus (200) according to claim 1, **characterized in that** the manipulating lever (222) has a manipulation changeover switch (227) for carrying out manipulation of another device (400).

16. A control apparatus (300) for controlling a manipulator apparatus (200) which comprises a motor and a rotatable device (240) rotatable on a first track axis and a second track axis, **characterized by** comprising:
a compare section (302) which compares change rates of position indicating values of turning movements around the first track axis and the second track axis with each other; and
a outputting power section (302) which outputs the greater change rate of position indicating values of turning movements around the first track axis and the second track axis to the motor.

17. A control apparatus (300) according to claim 16, **characterized in that** the compare section (302) compares a predetermined reference value to the greater change rate of position indicating values of turning movements around the first track axis and the second track axis, and outputs the greater value of them when the greater value of them is greater than the predetermined reference value.

18. A control method **characterized by** comprising:
instructing turning movement around a first axis and a second axis in association with a section to be controlled which moves around the first track axis and the second track axis;
comparing change rates of position indicating values of turning movements around the first track axis and the second track axis with each other; and
driving a motor according to the greater change rate of position indicating values of turning movements around the first track axis and the second track axis.

19. A control method according to claim 18,
**characterized in that** the greater change rate of position indicating values of turning movements around the first track axis and the second track axis is compared to a predetermined reference value, the greater value of them is outputted when the greater value of them is greater than the predetermined reference value, and the motor is driven according to the output.
